# NEUE EUROPÄISCHE PATENTSCHRIFT

(11) **EP 1 000 588 B2**
(45) Veröffentlichungstag und Bekanntmachung des Hinweises auf die Entscheidung über den Einspruch: **29.06.2016**
(45) Hinweis auf die Patenterteilung: 04.05.2005
(21) Anmeldenummer: 99121654.0
(22) Anmeldetag: 02.11.1999
(51) Int. Cl.: A61C 13/083, A61K 6/02

(54) **Keramische Dentalrestauration**
Ceramic dental restorations
Prothèse dentaire céramique

(30) Priorität: 13.11.1998 DE 19852516
(43) Veröffentlichungstag der Anmeldung: 17.05.2000
(73) Patentinhaber: DeguDent GmbH, 63457 Hanau-Wolfgang (DE)
(72) Erfinder: Steidl, Jürgen, 61206 Wöllstadt (DE); Assmann, Steffen, 61230 Bad Nauheim (DE)
(74) Vertreter: Stoffregen, Hans-Herbert

(56) Entgegenhaltungen:
- EP-A- 0 544 145
- EP-A- 0 759 289
- EP-A- 0 795 311
- WO-A-95/11866
- US-A- 4 604 366
- US-A- 4 798 536
- US-A- 5 705 273

## Beschreibung

Die Erfindung bezieht sich auf eine keramische Dentalrestauration bestehend aus einer Basiskeramik auf Basis einer leucithaltigen Glaskeramik, die mit einer Dentalkeramik verblendet ist, sowie ein Verfahren zur Herstellung einer keramischen Dentalrestauration.

Dentalrestaurationen wie künstliche Zähne, Kronen, Teilkronen, Brücken, Inlays, Onlays, Facetten, Stumpfaufbauten, Zahnwurzelkonstruktionen etc. werden überwiegend aus Metallegierungen, insbesondere auf Edelmetallbasis, gefertigt. Meist werden diese aus ästhetischen Gründen mit Keramik verblendet, um der Restauration, insbesondere im sichtbaren Bereich, ein dem natürlichen Zahn möglichst entsprechendes Aussehen zu geben.

Verblendkeramiken müssen in Schmelzpunkt und Wärmeausdehnungskoeffizient (WAK) sehr sorgfältig auf den Werkstoff des Grundgerüstes abgestimmt sein. Bei metallkeramischen Systemen wird üblicherweise der WAK der Keramik so gewählt, daß dieser geringfügig unter dem des metallischen Grundwerkstoffes liegt. Hierbei werden Zugspannungen in der Verblendkeramik verhindert und stattdessen Druckspannungen induziert, so daß beim Aufbrennen und Abkühlen sowie bei späteren Belastungen durch Temperaturwechsel das Auftreten von Sprüngen vermieden wird. Verblendkeramiken, die auf die'seit geraumen Jahren aufgrund besserer Vertäglichkeit dominierenden hochgoldhaltigen Dentallegierungen abgestimmt sind, besitzen typischerweise einen linearen WAK α_{(20-500°C)} von etwa 12,5·10⁻⁶ bis etwa 16,5·10⁻⁶ K⁻¹. Derartige keramische Werkstoffe sind beispielsweise in EP 0 478 937 beschrieben. Eine in der Praxis häufig eingesetzte Verblendkeramik dieser Kategorie weist einen WAK α_{(20-500°C)} von 15,0·10⁻⁶ K⁻¹ auf.

Als Alternative zu metallkeramischen Dentalrestaurationen werden vermehrt vollkeramische Systeme eingesetzt. Als Basiswerkstoffe werden hierbei überwiegend Glaskeramiken verwendet. Als Glaskeramiken werden Materialien bezeichnet, bei denen in einer Glasphase mindestens eine Kristallphase verteilt vorliegt. Glaskeramik kann erhalten werden, indem man ein amorphes Ausgangsglas einem gesteuerten partiellen Kristallisationsprozess unterwirft.

In beispielsweise US-A-4,798,536 werden vollkeramische Dentalrestaurationen aus Glaskeramik auf Feldspat-Basis beschrieben, wobei diese mindestens 45 Gew.-% Leucit als Kristallphase enthält. Hierbei wird das Dentalprodukt in Schlickertechnik geformt und dann gesintert.

Als besonders vorteilhaft für die Herstellung von vollkeramischen Dentalrestaurationen hat sich eine Technik erwiesen, bei der ein geeignetes dentalkeramisches Material unter Einwirkung von hoher Temperatur und unter Druck in den viskosen Zustand überführt und in eine der Dentalrestauration entsprechende Form verpreßt wird. Ein derartiges Material sowie das daraus hergestellte Dentalprodukt wird in der Dentaltechnik häufig auch als Preßkeramik bezeichnet. In EP 0 231 773 A1 wird diese Technik und ein hierfür geeigneter Preßofen beschrieben.

In DE 44 23 793 C1 wird eine als Preßkeramik verarbeitbare Dental-Glaskeramik beschrieben, die Leucit und mindestens eine weitere Kristallphase enthält.

In DE 44 23 794 C1 wird eine auch als Preßkeramik verarbeitbare Dental-Glaskeramik beschrieben, die als Kristallphase ZrO₂ und mindestens eine weitere Kristallphase enthält.

In DE 196 47 793 A1 wird eine als Preßkeramik verarbeitbare Dental-Glaskeramik beschrieben, die Lithiumdisilikat als Hauptkristallphase enthält.

Die bekannten und praktisch als Preßkeramik eingesetzten Glaskeramiken auf Leucitbasis weisen einen WAK α(_{20-500°C}) auf, der im Regelfall im Bereich von etwa 14·10⁻⁶ bis etwa 20·10⁻⁶ K⁻¹ liegt, und sollten daher mit den üblichen Verblendkeramiken mit niedriger liegendem WAK kombinierbar sein.

In der Praxis hat sich jedoch gezeigt, daß die Herstellung von vollkeramischen Dentalrestaurationen durch Kombination dieser bekannten Glaskeramiken als Gerüstwerkstoff mit den bei metallkeramischen Systemen verwendeten Verblendkeramiken trotz der gleichgearteten Abstimmung der Wärmeausdehnungskoeffizienten zu Problemen führt. So wird bei Verblendarbeiten eine hohe Ausschußrate durch Rißbildung beobachtet. Temperaturwechselbelastungen führen in nichtakzeptablem Maße zu weiterer Rißbildung und Versagen durch Bruch. Weiterhin wird bei den in der Praxis meist erforderlichen Mehrfachbränden ein Driften des WAK des Glaskeramikgrundkörpers zu höheren Werten hin beobachtet, was wiederum Sprungbildung und Bruch begünstigt.

Die EP-A-07953111 bezieht sich auf eine Dentalrestauration, bei der ein Gerüst aus Metall oder Keramik mit einer leucithaltigen Glaskeramik verblendet ist, deren Wärmeausdehnungskoeffizient in etwa 0,5 - 1,5·10⁻⁶ - K⁻¹ unter dem des Gerüsts liegt.

Der Erfindung liegt die Aufgabe zugrunde, eine keramische Dentalrestauration der eingangs genannten Art so weiterzubilden, dass bei der Verblendung mit üblichen Verblendkeramiken Rissbildung beim Abkühlen, Driften des Wärmeausdehnungskoeffizienten der Basiskeramik zu höheren Wärmeausdehnungskoeffizientwerten sowie Sprungbildung und Bruch unterbleiben.

Zur Lösung der Aufgabe wird erfindungsgemäß vorgeschlagen,
dass die Basiskeramik als Komoponenten

| | | |
|---|---|---|
| 40-95 | Gew.-% | SiO₂ |
| 5-25 | Gew.-% | Al₂O₃ |
| 5-25 | Gew.-% | K₂O |
| 0-25 | Gew.-% | Na₂O |
| 0-20 | Gew.-% | CaO |
| 0-8 | Gew.-% | B₂O₃ |
| 0-0,5 | Gew.-% | P₂O₅ |
| 0-3 | Gew.-% | F |

enthält, dass die Basiskeramik als einzige Kristallphase Leucit in einem Gesamtanteil von 20 bis 45 Gew.-% enthält, wobei mindestens 80% der theoretisch erzeugbaren Leucitmenge vorliegen, dass die Dentalkeramik einen linearen Wärmeausdehnungskoeffizienten α_{(20-500°C)} von 13,5·10⁻⁶ bis 17,0·10⁻⁶ K⁻¹ und die Basiskeramik einen linearen Wärmeausdehnungskoeffizient α_{(20-500°C)} von 12,5·10⁻⁶ bis 15,5·10⁻⁶ K⁻¹ aufweisen, wobei der Wärmeausdehnungskoeffizient der Basiskeramik um etwa 1,5·10⁻⁶ K⁻¹, unter dem der Verblendkeramik liegt.

Gegenstand der Erfindung ist somit eine keramische Dentalrestauration auf Basis einer wie vorstehend charakterisierten leucithaltigen Glaskeramik.

Gegenstand der Erfindung ist weiterhin ein Verfahren zur Herstellung einer keramischen Dentalrestauration auf Basis leucithaltiger Glaskeramik, bei dem man eine wie vorstehend charakterisierte Glaskeramik im viskosen Zustand in eine der Dentalrestauration entsprechende Form verpreßt.

Die Erfindung basiert auf der überraschenden Erkenntnis, daß es bei keramischen Dentalrestaurationen zur Vermeidung von Rißbildung und Folgeschäden vorteilhaft ist, den WAK der Basiskeramik, im Gegensatz zu der bewährten Situation bei metallkeramischen Systemen, niedrigerzu wählen als den der Verblendkeramik. Bei leucithaltiger Glaskeramik ist der WAK abhängig vom Gehalt an Leucit, wobei die reine Glasphase einen WAK α_{(20-500°C)} von ca. 10·10⁻⁶ K⁻¹ und der reine tetragonale Leucit einen WAK α_{(20-500°C)} von ca. 20·10⁻⁶ K⁻¹ aufweist. Leucit, K[AlSi₂O₆], kann sich durch Kristallisation in einem Ausgangsglas bilden, wenn dieses die Komponenten SiO₂, Al₂O₃ und K₂O enthält.

Die zur Herstellung der erfindungsgemäßen keramischen Dentalrestaurationen vorgesehene Glaskeramik enthält als Komponenten

| | | |
|---|---|---|
| 40-95 | Gew.-% | SiO₂ |
| 5-25 | Gew.-% | Al₂O₃ |
| 5-25 | Gew.-% | K₂O |
| 0-25 | Gew.-% | Na₂O |
| 0-20 | Gew.-% | CaO |
| 0-8 | Gew.-% | B₂O₃ |
| 0-0,5 | Gew.-% | P₂O₅ |
| 0-3 | Gew.-% | F. |

Die Glaskeramik kann als weitere Komponenten

| | | |
|---|---|---|
| 0-10 | Gew.-% | La₂O₃ |
| 0-10 | Gew.-% | Sb₂O₃ |
| 0-10 | Gew.-% | Li₂O |
| 0-20 | Gew.-% | MgO |
| 0-20 | Gew.-% | BaO |
| 0-20 | Gew.-% | SrO |
| 0-3,5 | Gew.-% | ZnO |
| 0-30 | Gew.-% | TiO₂ |
| 0-14 | Gew.-% | ZrO₂ |
| 0-30 | Gew.-% | CeO₂ |
| 0-30 | Gew.-% | SnO₂ |

enthalten.

Vorzugsweise besteht die Glaskeramik aus

| | | |
|---|---|---|
| 50-80 | Gew.-% | SiO₂ |
| 12-25 | Gew.-% | Al₂O₃ |
| 7-18 | Gew.-% | K₂O |
| 0,5-25 | Gew.-% | Na₂O |
| 0,1-2,5 | Gew.-% | CaO. |

Durch diese Zusammensetzung ist gewährleistet, daß die zu dem Dentalprodukt verarbeitete Glaskeramik als einzige Kristallphase Leucit in einem Gesamtanteil von 20 bis 45 Gew.-% enthält und daß sie somit einen linearen Wärmeausdehnungskoeffizient α_{(20-500°C)} von 12,5·10⁻⁶ bis 15,5·10⁻⁶ K⁻¹ aufweist. Hierbei liegen mindestens 80% der theoretisch erzeugbaren Leucitmenge in der Glaskeramik vor. Durch die nahezu quantitative Ausbildung der Leucitphase ist weiterhin gewährleistet, daß bei thermischen Folgebearbeitungen, wie insbesondere das Aufbringen von Verblendkeramik, keine wesentliche Änderung des Leucitgehaltes und damit kein Driften des WAK der Basisglaskeramik zu höheren WAK-Werten hin auftritt. Vorzugsweise enthält die Glaskeramik 35 bis 40 Gew.-% an Leucit, wobei sich dieses zu mindestens 90% der theoretisch erzeugbaren Menge gebildet hat; dieses Material besitzt dann einen WAK α_{(20-500°C)} von 13,0·10⁻⁶ bis 14,0·10⁻⁶ K⁻¹.

Mit einem derartigen Material können also gezielt dentale Basisglaskeramikobjekte hergestellt werden, deren WAK unter dem der üblichen dentalen Verblendkeramiken liegt und sich auch nicht zu höheren Werten hin verändert.

Die erfindungsgemäße keramische Dentalrestauration ist demnach vorzüglich für die Verblendung mit gängigen dentalen Verblendkeramiken mit linearem Wärmeausdehnungskoeffizient α_{(20-500°C)} von 13,5·10⁻⁶ bis 17,0·10⁻⁶ K⁻¹ geeignet. Hierbei wird der WAK der Basisglaskeramik so gewählt, daß er um etwa 1,5·10⁻⁶ K⁻¹ unter dem der Verblendkeramik liegt. Besonders bevorzugt ist eine derartige keramische Dentalrestauration, bei der der lineare Wärmeausdehnungskoeffizient α_{(20-500°C)} der Basiskeramik 13,0·10⁻⁶ bis 14,0·10⁻⁶ K⁻¹ und der lineare Wärmeausdehnungskoeffizient α_{(20-500°C)} der Verblendkeramik etwa 15,0·10⁻⁶ K⁻¹ beträgt.

Das Verfahren zur Herstellung der erfindungsgemäßen keramischen Dentalrestauration auf Basis leucithaltiger Glaskeramik erfolgt in der Weise, daß man
(a) ein Ausgangsglas oder eine Ausgangsglaskeramik in Form eines Pulvers oder eines Granulats herstellt, die als Komponenten

| | | |
|---|---|---|
| 40 - 95 | Gew.-% | SiO₂ |
| 5 - 25 | Gew.-% | Al₂O₃ |
| 5 - 25 | Gew.-% | K₂O |
| 0 - 25 | Gew.-% | Na₂O |
| 0 - 20 | Gew.-% | CaO |
| 0 - 8 | Gew.-% | B₂O₃ |
| 0 - 0,5 | Gew.-% | P₂O₅ |
| 0 - 3 | Gew.-% | F |

enthalten,
(b) aus dem Pulver oder Granulat einen Sinterkörper in Zylinder- oder Pelletform herstellt,
(c) den Sinterkörper bei einer Temperatur zwischen 850 und 1200 °C in den viskosen Zustand überführt und unter einem Druck zwischen 2 und 6 bar in eine der Dentalrestauration entsprechende Form verpreßt, wodurch sich eine Glaskeramik bildet, die als einzige Kristallphase Leucit in einem Gesamtanteil von 20 bis 45 Gew.-% enthält, wobei mindestens 80% der theoretisch erzeugbaren Leucitmenge vorliegen, und die einen linearen Wärmeausdehnungskoeffizient α_{(20-500°C)} von 12,5·10⁻⁶ bis 15,5·10⁻⁶ K⁻¹ aufweist.

Im Verfahrensschritt (a) werden die genannten Komponenten gemischt, zu einem Glas aufgeschmolzen und die Glasschmelze dann in Wasser gefrittet. Gewünschtenfalls können als weitere Komponenten

| | | |
|---|---|---|
| 0 - 10 | Gew.-% | La₂O₃ |
| 0 - 10 | Gew.-% | Sb₂O₃ |
| 0 - 10 | Gew.-% | Li₂O |
| 0 - 20 | Gew.-% | MgO |
| 0 - 20 | Gew.-% | BaO |
| 0 - 20 | Gew.-% | SrO |
| 0 - 3,5 | Gew.-% | ZnO |
| 0 - 30 | Gew.-% | TiO₂ |
| 0 - 14 | Gew.-% | ZrO₂ |
| 0 - 30 | Gew.-% | CeO₂ |
| 0 - 30 | Gew.-% | SnO₂ |

zugefügt werden. Vorzugsweise besteht das Ausgangsglas aus

| | | |
|---|---|---|
| 50 - 80 | Gew.-% | SiO₂ |
| 12 - 25 | Gew.-% | Al₂O₃ |
| 7 - 18 | Gew.-% | K₂O |
| 0,5 - 25 | Gew.-% | Na₂O |
| 0,1 - 2,5 | Gew.-% | CaO. |

Das durch Fritten erhaltene Glaspulver kann weiter aufgemahlen und auf eine bestimmte Teilchengröße, zweckmäßig ist 100 µm, klassiert werden. Zur Erzielung höchster Homogenität des Glases kann der Vorgang des Aufschmelzens, Frittens, Mahlens und Klassierens mehrfach wiederholt werden. Das Ausgangsglas weist nach diesen Vorgängen normalerweise noch keine Leucitphase auf. Durch etwaige anschließende thermische Behandlungen, etwa bei der Weiterverarbeitung zu Vor- bzw. Zwischenprodukten zur eigentlichen Herstellung von Dentalprodukten, kann aber durch partielle Kristallisation eine Umwandlung in eine Ausgangsglaskeramik erfolgen. Das Ausgangsglas bzw. die Ausgangsglaskeramik kann auch als Granulat vorliegen.

In Verfahrensschritt (b) wird aus dem Pulver oder Granulat des Ausgangsglases bzw. der Ausgangsglaskeramik ein Sinterkörper in Zylinder- oder Pelletform hergestellt. Hierzu wird das Material zu entsprechenden Formkörpern unter einem Druck von 40 bis 200 bar verpreßt und bei Temperaturen zwischen 750 und 950 °C über einen Zeitraum von 1 bis 5 Minuten gesintert. Im Hinblick auf die Anwendung als Preßkeramik werden die Sinterkörper zweckmäßigerweise als Ronden mit den Abmessungen 10 bis 12 mm Durchmesser und 8 bis 12 mm Höhe dimensioniert.

Im Verfahrensschritt (c) erfolgt die Verarbeitung des Sinterkörpers zu der geformten keramischen Dentalrestauration durch Verpressen im viskosen Zustand. Hierbei erfolgt die abschließende Umwandlung in eine Glaskeramik durch Kristallisation der Leucitphase. Die Verarbeitung kann im wesentlichen nach dem Verfahren und in einem Preßofen wie in EP 0 231 773 A1 beschrieben erfolgen. Die zunächst erforderliche Formherstellung für die Dentalrestauration erfolgt in der üblichen Technik der Wachsmodellation, Einbetten des mit einem Angußkanal versehenen Wachsmodells in eine handelsübliche selbsthärtende feuerfeste Einbettmässe und Austreiben des Wachses durch Erhitzen. Die Form wird in den Preßofen eingebracht, der entsprechend dimensionierte Angußkanal mit dem Sinterkörper aus Ausgangsglas bzw. Ausgangsglaskeramik bestückt, dieser bei einer Temperatur zwischen 950 und 1200 °C in den viskosen Zustand überführt und unter einem Druck zwischen 2 und 6 bar in die Form verpreßt. Bei diesem Vorgang erfolgt die Kristallisation von mindestens 80% der theoretisch erzeugbaren Leucitmenge, die dann als einzige Kristallphase in einem Gesamtanteil von 20 bis 45 Gew.-% in der Glaskeramik vorliegt. Die Glaskeramik weist hierdurch einen linearen Wärmeausdehnungskoeffizient α_{(20-500°C)} von 12,5·10⁻⁶ bis 15,5·10⁻⁶ K⁻¹ auf. Die erfindungsgemäße Glaskeramik läßt sich ohne weiteres bei einer Temperatur zwischen 900 und 1050 °C in den viskosen Zustand überführen und unter einem Druck zwischen 3 und 5 bar verpressen. Der Preßvorgang ist normalerweise nach einer Zeit von 20 Minuten abgeschlossen. Nach Abkühlen, Entformen und Entfernen des Angußstücks liegt die geformte keramische Dentalrestauration vor und kann dann den gegebenenfalls erforderlichen Folge- oder Abschlußbearbeitungen unterzogen werden. Hierzu zählt insbesondere die Verblendung mit einer Dentalkeramik, die einen WAK von 13,5·10⁻⁶ bis 17,0·10⁻⁶ K⁻¹ aufweist, wobei der WAK der Basiskeramik so gewählt wird, daß er 1,5·10⁻⁶ K⁻¹ unter dem der Verblendkeramik liegt.

Die erfindungsgemäße keramische Dentalrestauration erfüllt in vorzüglicher Weise alle Anforderungen, insbesondere hinsichtlich der mechanischen und optischen Eigenschaften, die an solche Produkte gestellt werden. Durch Variation der Komponenten der Glaskeramik innerhalb der angegebenen Bereiche können insbesondere die optischen Eigenschaften wie Färbung, Transluzenz und Opakizität beeinflußt werden.

Auch wenn sich die erfindungsgemäße Glaskeramik in idealer Weise für die Verarbeitung als Preßkeramik eignet, so kann sie aber auch mit entsprechendem Ergebnis nach anderen Techniken zu geformten Dentalprodukten verarbeitet werden. Solche Verarbeitungstechniken sind Formgebung in Schlickertechnik und Sinterung, Formguß der Glasschmelze, Formpressen und Sinterung, Gießen oder Sintern zu einem Rohling und anschließende mechanische Formgebung. Die Leucitkristallisation erfolgt in diesen Fällen in dem Sinter- bzw. einem Temperschritt.

### Beispiele 1 - 9

Die Komponenten gemäß nachfolgender **Tabelle 1** werden als Pulver homogen gemischt, bei einer Temperatur von 1550 - 1600 °C zu einem Glasfluß aufgeschmolzen und die Schmelzen in kaltem Wasser gefrittet. Die erhaltenen Glaspulver werden in einer Kugelmühle aufgemahlen und auf eine Partikelgröße von 100 µm gesiebt. Die Glaspulver werden durch Verpressen bei 100 bar und Sintern bei 850 °C über einen Zeitraum von 1 min. zu Sinterkörpern der Abmessungen 11.9 mm Durchmesser und 10 mm Höhe verarbeitet. Die Sinterkörper werden dann in einem Preßofen gemäß EP 0 231 773 A1 unter einem Preßdruck von 3 - 5 bar innerhalb von 1 min. zu Glaskeramik-Formkörpern (Probekörper) verarbeitet. **Tabelle 1** zeigt für die jeweilige Zusammensetzungen,die Preßtemperatur Tₚ und den WAK der resultierenden Glaskeramik. Der WAK der Glaskeramiken liegt zwischen 12,5·10⁻⁶ und 15,5·10⁻⁶ K⁻¹.

**Tabelle 1:**

| **Nr.** | **Zusammensetzung [Gew.-%]** | | | | | | | | | | **Tp (°C)** | **WAK α _{(20-500°C)} 10⁻⁶ K⁻¹]** |
|---|---|---|---|---|---|---|---|---|---|---|---|---|
| | **SiO₂** | **Al₂O₃** | **K₂O** | **Na₂O** | **Li₂O** | **B₂O₃** | **CaO** | **CeO₂** | **Sb₂O₃** | **F** | | |
| 1. | 65,3 | 15,7 | 14,7 | 0,9 | - | 0,9 | 1,6 | - | - | 0,9 | 1090 | 12,5 |
| 2. | 65,0 | 15,6 | 14,7 | 0,9 | 0,4 | 0,9 | 1,6 | - | - | 0,9 | 1060 | 14,3 |
| 3. | 64,8 | 16,5 | 15,3 | 1,9 | 0,5 | - | 0,7 | - | 0,2 | 0,1 | 1150 | 15,4 |
| 4. | 56,5 | 20,0 | 11,2 | 10,5 | - | 1,0 | 0,1 | 0,7 | - | - | 900 | 15,5 |
| 5. | 58,6 | 21,5 | 10,4 | 9,4 | - | - | 0,1 | - | - | - | 1100 | 15,3 |
| 6. | 58,1 | 22,0 | 9,4 | 10,4 | - | - | 0,1 | - | - | - | 1050 | 14,0 |
| 7. | 57,8 | 22,2 | 8,9 | 11,0 | - | - | 0,1 | - | - | - | 1020 | 13,1 |
| 8. | 59,6 | 22,2 | 9,5 | 8,6 | - | - | 0,1 | - | - | - | 1160 | 13,0 |
| 9. | 59,4 | 21,6 | 10,4 | 8,5 | - | - | 0,1 | - | - | - | 1120 | 15,3 |

### Beispiel 10

Sinterkörper mit Zusammensetzung gemäß **Beispiel 7** werden wie voran beschrieben bei unterschiedlichen Preßtemperaturen zu Dentalformkörpern (Kronen) verarbeitet. Danach werden die Kronen mit einer handelsüblichen Verblendkeramik (Duceragold®, Fa. Ducera) nach Herstellerangaben verblendet. Zum Test auf Temperaturwechselbeständigkeit werden die Kronen nach jedem Brand (insgesamt fünf Brände) aus kochendem Wasser in Wasser von Raumtemperatur (ΔT=80°C) abgeschreckt und auf Rißbildung untersucht. **Tabelle 2** zeigt den WAK der Dentalform-körper, der in Abhängigkeit von der Preßtemperatur zwischen 13,1·10⁻⁶ und 13,8·10⁻⁶ K⁻¹ liegt, nach dem Preßvorgang und die WAK-Differenz zur Verblendkeramik nach 1 - 5 Bränden, abhängig von der WAK-Veränderung (nach höheren Werten hin) der Verblendkeramik. Weiter zeigt **Tabelle 2** die Zahl der jeweiligen im Temperaturwechseltest untersuchten Kronen und die Zahl der hierbei defekten Kronen. Das Ergebnis des Temperaturwechseltest im ΔWAK-Bereich 0,5 - 2,5·10⁻⁶ K⁻¹ ist vorzüglich.

**Tabelle 2**

| **Preßtemperatur T_{P} [°C]** | **Preßkeramik WAK α_{(20-500°C)} [10⁻⁶K⁻¹] (driftstabil bei Folgebränden)** | **WAK-Differenz zur Verblendkeramik angegeben: Spanne zwischen 1. und 5. Brand** | **Getestete Kronen** | **davon Kronen mit Bruch (nach Anzahl der Brände)** |
|---|---|---|---|---|
| 1040 | 13,0 | 0,9 - 2,4 | 10 | 1 (nach 3. Brand) |
| 990 | 13,3 | 0,6 - 2,1 | 10 | 0 |
| 940 | 13,7 | 0,2 - 1,7 | 10 | 8 (nach 1. Brand) |
| 990 | 13,3 | 2,6 | 3. | 3 (nach 1. *) Brand) |

| | | | | |
|---|---|---|---|---|
| *) Vergleichgsversuch: Versuchscharge einer Verblendkeramik mit einem WAK α_{(20-500°C)} von 15,9. | | | | |

## Patentansprüche

1. Keramische Dentalrestauration bestehend aus einer Basiskeramik auf Basis einer leucithaltigen Glaskeramik, die mit einer Dentalkeramik verblendet ist, **dadurch gekennzeichnet, dass** die Basiskeramik als Komoponenten
| | | |
|---|---|---|
| 40-95 | Gew.-% | SiO₂ |
| 5-25 | Gew.-% | Al₂O₃ |
| 5-25 | Gew.-% | K₂O |
| 0-25 | Gew.-% | Na₂O |
| 0-20 | Gew.-% | CaO |
| 0-8 | Gew.-% | B₂O₃ |
| 0-0,5 | Gew.-% | P₂O₅ |
| 0-3 | Gew.-% | F |
enthält. dassdie Basiskeramik als einzige Kristallphase Leucit in einem Gesamtanteil von 20 bis 45 Gew.-% enthält. wobei mindestens 80% der theoretisch erzeugbaren Leucitmenge vorliegen, dass die Dentalkeramik einen linearen Wärmeausdehnungskoeffizienten α_{(20-500°C)} von 13.5·10⁻⁶ bis 17,0·10⁻⁶ K⁻¹ und die Basiskeramik einen linearen Wärmeausdehnungskoeffizient α_{(20-500°C)} von 12,5·10⁻⁶ bis 15,5·10⁻⁶ K⁻¹ aufweisen, wobei der Wärmeausdehnungskoeffizientder Basiskeramik um etwa 1,5·10⁻⁶ K⁻¹ unter dem der Verblendkeramik liegt.

2. Keramische Dentalrestauration nach Anspruch 1, **dadurch gekennzeichnet, daß** die Glaskeramik als weitere Komponenten
| | | |
|---|---|---|
| 0-10 | Gew.-% | La₂O₃ |
| 0-10 | Gew.-% | Sb₂O₃ |
| 0-10 | Gew.-% | Li₂O |
| 0-20 | Gew.-% | MgO |
| 0-20 | Gew.-% | BaO |
| 0-20 | Gew.-% | SrO |
| 0-3,5 | Gew.-% | ZnO |
| 0-30 | Gew.-% | TiO₂ |
| 0-14 | Gew.-% | ZrO₂ |
| 0-30 | Gew.-% | CeO₂ |
| 0-30 | Gew.-% | SnO₂ |
enthält.

3. Keramische Denlalrestauration nach Anspruch 1 oder 2, **dadurch gekennzeichnet, daß** die Glaskeramik aus
| | | |
|---|---|---|
| 50-80 | Gew.-% | SiO₂ |
| 12-25 | Gew.-% | Al₂O₃ |
| 7-18 | Gew.-% | K₂O |
| 0,5-25 | Gew.-% | Na₂O |
| 0,1-2,5 | Gew.-% | CaO |
besteht.

4. Keramische Dentalrestauration nach Anspruch 3, **dadurch gekennzeichnet, daß** der lineare Wärmeausdehnungskoeffizient α_{(20-500°C)} der Basiskeramik 13,0·10⁻⁶ bis 14,0·10⁻⁶ K⁻¹ und der lineare Wärmeausdehnungskoeffizient α_{(20-500°C)} der Verblendkeramik etwa 15,0·10⁻⁶ K⁻¹ beträgt.

5. Verfahren zur Herstellung einer keramischen Dentalrestauration gemäß denen der Ansprüche 1 bis 4, **dadurch gekennzeichnet, dass** man
(a) ein Ausgangsglas oder eine Ausgangsglaskeramik in Form eines Pulvers oder eines Granulats herstellt, die als Komponenten
| | | |
|---|---|---|
| 40-95 | Gew.-% | SiO₂ |
| 5-25 | Gew.-% | Al₂O₃ |
| 5-25 | Gew.-% | K₂O |
| 0-25 | Gew.-% | Na₂O |
| 0-20 | Gew.-% | CaO |
| 0-8 | Gew.-% | B₂O₃ |
| 0-0,5 | Gew.-% | P₂O₅ |
| 0-3 | Gew.-% | F |
enthalten.
(b) aus dem Pulver oder Granulat einen Sinterkörper in Zylinder- oder Pelletform herstellt.
(c) den Sinterkörper bei einer Temperatur zwischen 850 und 1200 °C in den viskosen Zustand überführt und unter einem Druck zwischen 2 und 6 bar In eine der Dentalrestauration entsprechende Form verpreßt, wodurch sich eine Glaskeramik bildet, die als einzige Kristallphase Leucit in einem Gesamtanteil von 20 - 45 Gew.-% enthält, wobei mindestens 80% der theoretisch erzeugbaren Leucitmenge vorliegen, und die einen linearen Wärmeausdehnungskoeffizient α_{(20-500°C)} von 12,5·10⁻⁶ bis 15,5·10⁻⁶ K⁻¹ aufweist.
dass man die Dentalrestauration mit der Dentalkeramik, die einen linearen Wärmeausdehnungskoeffizient α_{(20-500°C)} von 13,5 · 10⁻⁶ bis 17,0·10⁻⁶ K⁻¹ aufweist, verblendet. wobei der Wärmeausdehnungskoeffizient der Basiskeramik so gewählt wird, daß er um etwa 1,5·10⁻⁶ K⁻¹, unter dem der Verblendkeramik liegt.

6. Verfahren nach Anspruch 5, **dadurch gekennzeichnet, daß** man ein Ausgangsglas oder eine Ausgangsglaskeramik verwendet, die als weitere Komponenten
| | | |
|---|---|---|
| 0-10 | Gew.-% | La₂O₃ |
| 0-10 | Gew.-% | Sb₂O₃ |
| 0-20 | Gew.-% | Li₂O |
| 0-20 | Gew.-% | MgO |
| 0-20 | Gew.-% | BaO |
| 0-20 | Gew.-% | SrO |
| 0-3,5 | Gew.-% | ZnO |
| 0-30 | Gew.-% | TiO₂ |
| 0-14 | Gew.-% | ZrO₂ |
| 0-30 | Gew.-% | CeO₂ |
| 0-30 | Gew.-% | SnO₂ |
enthalten.

7. Verfahren nach Anspruch 5 oder 6, **dadurch gekennzeichnet, daß** man ein Ausgangsglas oder eine Ausgangsglaskeramik verwendet, die aus
| | | |
|---|---|---|
| 50-80 | Gew.-% | SiO₂ |
| 12-25 | Gew.-% | Al₂O₃ |
| 7-18 | Gew.-% | K₂O |
| 0,5-25 | Gew.-% | Na₂O |
| 0,1-2,5 | Gew.-% | CaO |
bestehen.

8. Verfahren nach den Ansprüchen 5 bis 7, **dadurch gekennzeichnet, daß** man den Sinterkörper bei einer Temperatur zwischen 900 und 1050°C in den viskosen Zustand überführt und unter einem Druck zwischen 3 und 5 bar ineine der Dentalrestauration entsprechende Form verpreßt.

9. Verfahren nach den Ansprüchen 5 bis 8, **dadurch gekennzeichnet, daß** man eine Basiskeramik mit linearem Wärmeausdehnungskoeffizient α_{(20-500°C)} von 13,0·10⁻⁶ bis 14,0·10⁻⁶ K⁻¹ mit einer Verblendkeramik mit linearem Wärmeausdehnungskoeffizient α_{(20-500°C)} von etwa 15,0·10⁻⁶ K⁻¹ verblendet.

## Claims

1. Ceramic dental restoration composed of a base ceramic based on a leucite-containing glass ceramic that is faced with a dental ceramic, **characterized in that** the base ceramic contains as components
| | | |
|---|---|---|
| 40-95 | wt.% | SiO₂ |
| 5-25 | wt.% | Al₂O₃ |
| 5-25 | wt.% | K₂O |
| 0-25 | wt.% | Na₂O |
| 0-20 | wt.% | CaO |
| 0-8 | wt.% | B₂O₃ |
| 0-0.5 | wt.% | P₂O₅ |
| 0-3 | wt.% | F |
in that the base ceramic contains, as sole crystalline phase, leucite in a total proportion from 20 to 45 wt.% , wherein at least 80 % of the theoretically producible quantity of leucite is present, **in that** the dental ceramic exhibits a linear coefficient of thermal expansion α_{(20-500°C)} from 13.5·10⁻⁶ to 17.0·10⁻⁶ K⁻¹ and the base ceramic exhibits a linear coefficient of thermal expansion α_{(20-500°C)} from 12.5·10⁻⁶ to 15.5·10⁻⁶ K⁻¹, wherein the coefficient of thermal expansion of the base ceramic is about 1.5·10⁻⁶ K⁻¹ below that of the facing ceramic.

2. Ceramic dental restoration according to claim 1, **characterized in that** the glassceramic contains as further components
| | | |
|---|---|---|
| 0-10 | wt.% | La₂O₃ |
| 0-10 | wt.% | Sb₂O₃ |
| 0-10 | wt.% | Li₂O |
| 0-20 | wt.% | MgO |
| 0-20 | wt.% | BaO |
| 0-20 | wt.% | SrO |
| 0-3.5 | wt.% | ZnO |
| 0-30 | wt.% | TiO₂ |
| 0-14 | wt.% | ZrO₂ |
| 0-30 | wt.% | CeO₂ |
| 0-30 | wt.% | SnO₂ |

3. Ceramic dental restoration according to claim 1 or 2, **characterized in that** the glassceramic consists of
| | | |
|---|---|---|
| 50-80 | wt.% | SiO₂ |
| 12-25 | wt.% | Al₂O₃ |
| 7-18 | wt.% | K₂O |
| 0.5-25 | wt.% | Na₂O |
| 0.1-2.5 | wt.% | CaO |

4. Ceramic dental restoration according to claim 3, **characterized in that** the linear coefficient of thermal expansion α_{(20-500°C)} of the base ceramic amounts to 13.0·10⁻⁶ to 14.0·10⁻⁶ K⁻¹ and the linear coefficient of thermal expansion α_{(20-500°C)} of the facing ceramic amounts to about 15.0·10⁻⁶ K⁻¹.

5. Process for manufacturing a ceramic dental restoration according to the ones of claims 1 to 4, **characterized in that**
(a) an initial glass or an initial glass ceramic is produced in the form of a powder or a granulate which contains as components
| | | |
|---|---|---|
| 40-95 | wt.% | SiO₂ |
| 5-25 | wt.% | Al₂O₃ |
| 5-25 | wt.% | K₂O |
| 0-25 | wt.% | Na₂O |
| 0-20 | wt.% | CaO |
| 0-8 | wt.% | B₂O₃ |
| 0-0.5 | wt.% | P₂O₅ |
| 0-3 | wt.% | F |
(b) a sintered compact in cylinder or pellet form is produced from the powder or granulate,
(c) the sintered compact is converted into the viscous state at a temperature between 850 and 1,200 °C and is press-molded under a pressure between 2 and 6 bar into a shape corresponding to the dental restoration, as a result of which a glass ceramic is formed which contains as sole crystalline phase leucite in a total proportion of 20 - 45 wt.%, wherein at least 80 % of the theoretically producible quantity of leucite are present, and which exhibits a linear coefficient of thermal expansion α_{(20-500°C)} from 12.5·10⁻⁶ to 15.5·10⁻⁶ K⁻¹,
**in that** the dental restoration is faced with the dental ceramic which exhibits a linear coefficient of thermal expansion α_{(20-500°C)} from 13.5·10⁻⁶ to 17.0·10⁻⁶ K⁻¹, wherein the coefficient of thermal expansion of the base ceramic is chosen so that it is about 1.5·10⁻⁶ K⁻¹ below that of the facing ceramic.

6. Process according to claim 5, **characterized in that** an initial glass or an initial glass ceramic is used which contains as further components
| | | |
|---|---|---|
| 0-10 | wt.% | La₂O₃ |
| 0-10 | wt.% | Sb₂O₃ |
| 0-10 | wt.% | Li₂O |
| 0-20 | wt.% | MgO |
| 0-20 | wt.% | BaO |
| 0-20 | wt.% | SrO |
| 0-3.5 | wt.% | ZnO |
| 0-30 | wt.% | TiO₂ |
| 0-14 | wt.% | ZrO₂ |
| 0-30 | wt.% | CeO₂ |
| 0-30 | wt.% | SnO₂ |

7. Process according to claim 5 or 6, **characterized in that** an initial glass or an initial glass ceramic is used which consists of
| | | |
|---|---|---|
| 50-80 | wt.% | SiO₂ |
| 12-25 | wt.% | Al₂O₃ |
| 7-18 | wt.% | K₂O |
| 0.5-25 | wt.% | Na₂O |
| 0.1-2.5 | wt.% | CaO |

8. Process according to claims 5 to 7, **characterized in that** the sintered compact is converted into the viscous state at a temperature between 900 and 1,050 °C and is press-molded under a pressure between 3 and 5 bar into a shape corresponding to the dental restoration.

9. Process according to claims 5 to 8, **characterized in that** a base ceramic having a linear coefficient of thermal expansion α_{(20-500°C)} from 13.0·10⁻⁶ to 14.0·10⁻⁶ K⁻¹ is faced with a facing ceramic having a linear coefficient of thermal expansion α_{(20-500°C)} of about 15.0·10⁻⁶ K⁻¹.

## Revendications

1. Prothèse dentaire céramique constituée d'une céramique de base à base d'une vitrocéramique contenant de la leucite, qui est revêtue d'une céramique dentaire,
**caractérisée en ce que**
la céramique de base contient comme composants :
- 40 à 95 % en poids de SiO₂
- 5 à 25 % en poids de Al₂O₃
- 5 à 25 % en poids de K₂O
- 0 à 25 % en poids de Na₂O
- 0 à 20 % en poids de CaO
- 0 à 8 % en poids de B₂O₃
- 0 à 0,5 % en poids de P₂O₅
- 0 à 3 % en poids de F
la céramique de base contient de la leucite, en tant que phase cristalline unique, dans une proportion totale allant de 20 à 45 % en poids, avec au moins 80 % de la quantité de leucite pouvant être théoriquement obtenue, la céramique dentaire présente un coefficient de dilatation thermique linéaire α_{(20-500°C)} de 13,5·10⁻⁶ à 17,0·10⁻⁶ K⁻¹ et la céramique de base présente un coefficient de dilatation thermique linéaire α_{(20-500°C)} de 12,5·10⁻⁶ à 15,5·10⁻⁶ K⁻¹, le coefficient de dilatation thermique de la céramique de base se situant à environ 1,5·10⁻⁶ K⁻¹, au-dessous de celui de la céramique de revêtement.

2. Prothèse dentaire céramique selon la revendication 1,
**caractérisée en ce que**
la vitrocéramique contient comme autres composants :
- 0 à 10 % en poids de La₂O₃,
- 0 à 10 % en poids de Sb₂O₃,
- 0 à 10 % en poids de Li₂O,
- 0 à 20 % en poids de MgO,
- 0 à 20 % en poids de BaO,
- 0 à 20 % en poids de SrO,
- 0 à 3,5 % en poids de ZnO,
- 0 à 30 % en poids de TiO₂,
- 0 à 14 % en poids de ZrO₂,
- 0 à 30 % en poids de CeO₂,
- 0 à 30 % en poids de SnO₂,

3. Prothèse dentaire céramique selon la revendication 1 ou 2,
**caractérisée en ce que**
la vitrocéramique est constituée de :
- 50 à 80 % en poids de SiO₂,
- 12 à 25 % en poids de Al₂O₃,
- 7 à 18 % en poids de K₂O,
- 0,5 à 25 % en poids de Na₂O,
- 0,1 à 2,5 % en poids de CaO.

4. Prothèse dentaire céramique selon la revendication 3,
**caractérisée en ce que**
le coefficient de dilatation thermique linéaire α_{(20-500°C)} de la céramique de base s'élève de 13,0·10⁻⁶ à 14,0·10⁻⁶ K⁻¹, et le coefficient de dilatation thermique linéaire α_{(20-500°C)} de la céramique de revêtement s'élève à environ 15,0·10⁻⁶ K⁻¹.

5. Procédé pour fabriquer une prothèse dentaire céramique selon l'une des revendications 1 à 4,
**caractérisé en ce que**
(a) on fabrique un verre de départ ou une vitrocéramique de départ sous forme de poudre ou de granulat, qui contient comme composants :
- 40 à 95 % en poids de SiO₂,
- 5 à 25 % en poids de Al₂O₃,
- 5 à 25 % en poids de K₂O,
- 0 à 25 % en poids de Na₂O,
- 0 à 20 % en poids de CaO,
- 0 à 8 % en poids de B₂O₃,
- 0 à 0,5 % en poids de P₂O₅,
- 0 à 3 % en poids de F,
(b) à partir de la poudre ou du granulat, on fabrique un corps fritté sous forme de cylindre ou de pastille,
(c) on réduit le corps fritté à une température comprise entre 850 et 1200 °C à l'état visqueux et on l'injecte à une pression comprise entre 2 et 6 bars dans une forme correspondant à la prothèse dentaire, une vitrocéramique se formant par ce moyen, qui contient de la leucite en tant que phase cristalline unique dans une proportion totale de 20 à 45 % en poids, avec au moins 80 % de la quantité de leucite pouvant être théoriquement obtenue, et qui présente un coefficient de dilatation thermique linéaire α_{(20-500°C)} de 12,5·10⁻⁶ à 15,5·10⁻⁶ K⁻¹,
on revêt la prothèse dentaire avec la céramique dentaire qui présente un coefficient de dilatation thermique linéaire α_{(20-500°C)} de 13,5·10⁻⁶ à 17,0·10⁻⁶ K⁻¹, le coefficient de dilatation thermique de la céramique de base étant choisi de telle sorte qu'il soit à environ 1,5·10⁻⁶ K⁻¹, au-dessous de celui de la céramique de revêtement.

6. Procédé selon la revendication 5,
**caractérisé en ce qu'**
on utilise un verre de départ ou une vitrocéramique de départ, qui contient comme autres composants :
- 0 à 10 % en poids de La₂O₃,
- 0 à 10 % en poids de Sb₂O₃,
- 0 à 10 % en poids de Li₂O,
- 0 à 20 % en poids de MgO,
- 0 à 20 % en poids de BaO,
- 0 à 20 % en poids de SrO,
- 0 à 3,5 % en poids de ZnO,
- 0 à 30 % en poids de TiO₂,
- 0 à 14 % en poids de ZrO₂,
- 0 à 30 % en poids de CeO₂,
- 0 à 30 % en poids de SnO₂.

7. Procédé selon la revendication 5 ou 6,
**caractérisé en ce qu'**
on utilise un verre de départ ou une vitrocéramique de départ, qui est constitué(e) de :
- 50 à 80 % en poids de SiO₂,
- 12 à 25 % en poids de Al₂O₃,
- 7 à 18 % en poids de K₂O,
- 0,5 à 25 % en poids de Na₂O,
- 0,1 à 2,5 % en poids de CaO.

8. Procédé selon les revendications 5 à 7,
**caractérisé en ce qu'**
on réduit le corps fritté à une température comprise entre 900 et 1050 °C à l'état visqueux et on l'injecte à une pression comprise entre 3 et 5 bars dans une forme correspondant à la prothèse dentaire.

9. Procédé selon les revendications 5 à 8,
**caractérisé en ce qu'**
on revêt une céramique de base ayant un coefficient de dilatation thermique linéaire α_{(20-500°C)} de 13,0·10⁻⁶ à 14,0·10⁻⁶ K⁻¹ avec une céramique de revêtement ayant un coefficient de dilatation thermique linéaire α_{(20-500°C)} d'environ 15,0·10⁻⁶ K⁻¹.
